# NEW EUROPEAN PATENT SPECIFICATION

(11) **EP 0 059 506 B2**
(45) Date of publication and mention of the opposition decision: **26.01.1994**
(45) Mention of the grant of the patent: 24.06.1987
(21) Application number: 82200209.3
(22) Date of filing: 22.02.1982
(51) Int. Cl.: A61F 13/15, B29C 51/10, B29C 59/06

(54) **Macroscopically expanded three-dimensional plastic web exhibiting non-glossy visible surface and cloth-like tactile impression and process for its manufacture**
Dreidimensional makroskopisch verstreckte Kunststoffbahn mit einer nicht glänzenden sichtbaren Oberfläche und einem stoffartigen Griff und Verfahren zu ihrer Herstellung
Bande en matière plastique étirée de manière macroscopique dans trois dimensions présentant une surface visible non-brillante et un toucher ressemblant l'étoffe et procédé pour sa fabrication

(30) Priority: 02.03.1981 US 239875
(43) Date of publication of application: 08.09.1982
(73) Proprietor: THE PROCTER & GAMBLE COMPANY, Cincinnati Ohio 45202 (US)
(72) Inventor: Ahr, Nicholas Albert, Cincinnati Ohio 45239 (US); Mullane, William Irvin, Jr., Cincinnati Ohio 45215 (US); Louis, Paul Raymond, Cincinnati Ohio 45211 (US); Ouellette, William Robert, Cincinnati Ohio 45240 (US)
(74) Representative: Bottema, Johan Jan

(56) References cited:
- EP-A- 0 018 684
- DE-A- 3 016 568
- US-A- 23 910
- US-A- 2 689 379
- US-A- 3 950 480
- US-A- 4 259 286

## Description

### Technical Field

The present invention has relation to resilient plastic webs exhibiting many of the three-dimensional, cloth-like properties and characteristics previously obtainable only in fibrous webs.

The present invention has further relation to resilient plastic webs which exhibit a combination of desirable, but previously incompatible attributes of prior art fibrous webs and prior art plastic webs in a single structure without deleterious side effects.

The present invention has further relation to plastic webs having a three-dimensional macroscopic pattern and having at least one substantially non-glossy visible surface and a cloth-like or fiber-like tactile impression.

### Background Art

It has long been known in the disposable absorbent bandage art that it is extremely desirable to construct absorptive devices, such as disposable diapers, sanitary napkins, incontinent devices, absorbent wound dressings, and the like, presenting a dry surface feel to the user to improve wearing comfort and to minimize the development of undesirable skin conditions due to prolonged exposure to moisture absorbed within the bandage. One viable prior art solution to the aforementioned problem is disclosed in U.S. Patent 4,041,951 issued to Sanford on August 16, 1977.

The Sanford patent discloses a preferred disposable diaper structure comprising a substantially planar, moisture absorbent layer disposed between a soft topsheet and a moisture-resistant backing sheet. The nonwoven fibrous topsheet preferably comprises an integral structure containing a multiplicity of depressed areas which intimately contact the uppermost surface of a substantially planar, moisture absorbent layer. The nondepressed areas of the topsheet contact the wearer's skin in-use. In a particularly preferred embodiment, the nonwoven fibrous topsheet is comprised of a substantially hydrophobic material exhibiting wet resilience such that the topsheet tends to resume its substantially three-dimensional character upon removal of pressure applied against the topsheet by the body movements of the wearer. The nondepressed areas of the topsheet, which are of substantially the same density as the depressed areas, tend to isolate the wearer's skin from moisture contained within the moisture absorbent layer, thereby providing surface dryness and resistance to rewetting when the structure is temporarily subjected to pressure resulting from the wearer's body movements.

U.S. Patent 3,814,101 issued to Kozak on June 4, 1974, attacks the problem of a wet topsheet in a manner slightly different from the use of hydrophobic nonwoven materials. Kozak suggests a topsheet of a nonfibrous, hydrophobic film which is provided with a plurality of valvular slits which restrict the reverse flow of liquid from the absorbent element of the device.

U.S. Patent 3,929,135 issued to Thompson on December 30, 1975, suggests a macroscopically expanded three-dimensional topsheet comprised of liquid-impermeable material, but provided with tapered capillaries, said capillaries having a base opening in the plane of the topsheet and an apex opening remote from the plane of the topsheet, said apex opening being in intimate contact with the absorbent pad utilized in the disposable absorbent bandage.

As utilized herein, the term "macroscopically expanded", when used to describe three-dimensional plastic webs, ribbons and films, refers to webs, ribbons and films which have been caused to conform to the surface of a three-dimensional forming structure so that both surfaces thereof exhibit the three-dimensional pattern of said forming structure, said pattern being readily visible to the naked eye when the perpendicular distance between the viewer's eye and the plane of the web is about 30 cm (12 inches). Such macroscopically expanded webs, ribbons and films are typically caused to conform to the surface of said forming structures by embossing, i.e., when the forming structure exhibits a pattern comprised primarily of male projections, by debossing, i.e., when the forming structure exhibits a pattern comprised primarily of female capillary networks, or by extrusion of a resinous melt directly onto the surface of a forming structure of either type. By way of contrast, the term "planar", when utilized herein to describe plastic webs, ribbons and films, refers to the overall condition of the web, ribbon or film when viewed by the naked eye on a macroscopic scale. In this context "planar" webs, ribbons and films may include webs, ribbons and films having fine scale surface aberrations on one or both sides, said surface aberrations not being readily visible to the naked eye when the perpendicular distance between the viewer's eye and the plane of the web is about 30 cm (12 inches) or greater. Planar plastic webs of the aforementioned type are known in the art.

The topsheet disclosed in the aforementioned Thompson patent allows the free transfer of fluids from the body into the absorbent element of the device while inhibiting the reverse flow of these fluids. This provides a relatively much drier surface in contact with the user than had been previously obtainable. However, experience has demonstrated that despite the highly effective fluid transfer and fluid isolation characteristics exhibited by plastic topsheets of the type generally disclosed in the Thompson patent and their proven compatibility with the wearer's skin, many users find it psychologically undesirable to employ a material which is perceivably plastic in contact with their skin. It is believed that this user reaction is due partly to the highly regulated tapered capillary pattern on the wearer-contacting surface of the topsheet and partly to the glossy appearance of the plastic material.

The commonly assigned, co-pending European patent application No. 80200156.0 Publication No. 0018020, discloses an improved macroscopically expanded three-dimensional plastic web which eliminates the regulated pattern of tapered capillaries, as disclosed in U.S. Patent 3,929,135, while preserving the desirable fluid transport properties of the structure.

The macroscopically expanded three-dimensional plastic web disclosed in Application No. 80200156.0 (EP-A-18020) preferably exhibits a fine scale three-dimensional microstructure comprising a regulated continuum of capillary networks, preferably of steadily decreasing size, originating in and extending from one surface of said web and preferably terminating in the form of apertures in the opposite surface thereof to promote rapid liquid transport in the direction of decreasing capillary size. The web's fiber-like appearance is comprised of a continuum of fiber-like elements, each end of said fiber-like elements being interconnected to at least one other of said fiber-like elements. In a particularly preferred embodiment, the interconnected fiber-like elements are substantially non-aligned with respect to one another.

A typical capillary network in the structure of Application No. 80200156.0 (EP-A-18020) comprises an uppermost capillary opening formed by a multiplicity of primary fiber-like elements interconnected to one another in the uppermost plane of the web. The opening may, if desired, be further subdivided into smaller capillary openings by secondary and tertiary fiber-like elements at planes located below the wearer-contacting surface of the web.

Each of the primary fiber-like elements exhibits a substantially uniform U-shaped cross-section along its length. Its cross-section comprises a base portion located in the wearer-contacting plane and a primary sidewall portion joined to each edge of said primary base portion and extending generally in the direction of the absorbent pad-contacting surface of the web. The secondary and tertiary fiber-like elements, when employed, are generally similar, but originate in planes below the wearer-contacting surface of the web. Because the plastic web of Application No. 80200156.0 (EP-A-18020) is comprised of a multiplicity of interconnected fiber-like elements rather than a continuous, regulated pattern of tapered capillaries, as disclosed in U.S. Patent 3,929,135, its appearance and tactile impression are generally perceived as more fiber-like.

In a particularly preferred embodiment of the invention of Application No. 80200156.0 (EP-A-18020), the web's visible surface (i.e., that surface which is generally observable from a perspective which is substantially perpendicular to the plane of the web) is also provided with a fine scale texture comprising a multiplicity of generally parallel V-shaped grooves to create a non-planar surface appearance in the web. The application suggests that the ridges and valleys formed in the plastic web by the V-shaped grooves in the forming structure tend to reduce the web's gloss.

The commonly assigned European Patent Application No. 80200365.7 filed April 22, 1980, Publication No. 18,684, likewise recognizes the perceived drawbacks associated with a glossy-appearing macroscopically expanded three-dimensional plastic web to be utilized in contact with the skin. Accordingly, European Patent Application No. 80200365.7 (EP-A-18684) discloses a macroscopically expanded three-dimensional plastic film provided with a surface texturing treatment. In a particularly preferred embodiment, the film is employed as a diaper topsheet. The texturing treatment preferably provides a multiplicity of "nubbles" integrally formed on at least one surface to "improve the tactile impression of the thermoplastic film and to reduce its gloss". The nubbles, which impart an irregular and unsmooth texture to the wearer-contacting surface of the topsheet, are small protuberances which project outwardly from the wearer-contacting surface of the topsheet. The number, size, and spacing of the nubbles may be varied within a critical range to give differing degrees of irregularity to the wearer-contacting surface. The nubbles are preferably spherical or spheroidal in cross-section, although it is suggested that other cross-sectional shapes may be used.

A preferred method for manufacturing such a topsheet is disclosed in the commonly assigned, U.S. Patent No. 4,259,286 to Paul R. Louis, Eugene R. Sorenson and Thomas R. Ballard entitled METHOD AND APPARATUS FOR TEXTURING A THERMOPLASTIC FILM, issued March 31, 1981.

Briefly, Louis et al. employs a perforate tubular forming member having a multiplicity of particles affixed to its outermost or web-contacting surface. In a particularly preferred embodiment, the tubular member is coated with a mixture of said particles and epoxy, which epoxy may be electro-statically sprayed onto said tubular member and thereafter cured. As should be apparent from the foregoing description, the variability in size and spacing of the particles which form the nubbles in the plastic film processed on said tubular forming member is completely random, and will depend upon such factors as the uniformity in size and shape of the particles employed, the ratio of particles to epoxy, and the uniformity of the spraying techniques.

While the striation treatment disclosed in the European Application No. 80200156.0 (EP-A-18020) and the random particle treatment disclosed in U.S. Patent No. 4,259,286 and European Application No. 80200365.7 (EP-A-18684) have in certain instances been employed to produce macroscopically expanded three-dimensional plastic webs exhibiting reduced gloss when compared to untreated prior art plastic webs, it is nonetheless an object of the present invention to accurately define the relevant criteria which must be satisfied to ensure that macroscopically expanded three-dimensional plastic webs which satisfy said criteria will exhibit a substantially non-glossy visible surface when the perpendicular distance between the viewer's eye and the plane of the web is about twelve inches or greater.

It is another object of the present invention to accurately define the relevant criteria which must be satisfied to ensure that macroscopically expanded three-dimensional plastic webs which satisfy said criteria will exhibit a substantially non-glossy visible surface and an improved cloth-like or fiber-like tactile impression.

It is another object of the present invention to provide macroscopically expanded, three-dimensional plastic webs which do in fact satisfy the aforementioned criteria.

### Disclosure of the Invention

According to the present invention there is provided a plastic web having a three dimensional macroscopic pattern and having a visible surface exhibiting microscopic surface aberrations which are not discernible to the naked eye when the perpendicular distance between the viewers eye and the plane of said web is at least 30cm, each of said surface aberrations being free of planar areas which are large enough to inscribe a 0.1mm diameter circle, whereby light incident upon said visible surface is diffusely reflected into a multiplicity of directions by said surface aberrations to reduce the gloss on said surface, characterised in that said surface aberrations are regularly sized and are disposed in a regularly spaced pattern over substantially all of said visible surface, said aberration size being measured in the plane in which surface aberrations originate, said spaced pattern being such that the diameter of any circle which can be inscribed on any planar surface of the web intermediate adjacent surface aberrations is less than 0.1mm, the minimum average amplitude of said surface aberrations being 0.005 mm, as measured perpendicular to and from the surface in which said surface aberrations originate, the maximum dimension of any said surface aberrations being less than 0.15mm as measured substantially perpendicular to the amplitude of said surface aberrations, in the plane in which said surface aberrations originate whereby said visible surface is rendered substantially non glossy.

Since the microscopic pattern of surface aberrations responsible for causing the diffuse reflection is not visually perceived by the observer under normal conditions, i.e., when the perpendicular distance between the viewer's eye and the plane of the web is 30 cm or greater, macroscopically expanded three-dimensional plastic webs of the present invention are visually perceived as more cloth-like or fiber-like. The surface aberrations employed in the practice of the present invention may comprise protuberances projecting generally outwardly from the surface of said web or depressions projecting generally inwardly from the surface of said web.

Plastic webs of the present invention may also be made to exhibit a more cloth-like or fiber-like tactile impression by employing surface aberrations having a minimum average amplitude of at least 0.2 mils (i.e., 0.005 mm), as measured from the top of said protuberance or the bottom of said depression, as the case may be, to the plane in which said aberration originates.

Preferred methods for forming plastic webs according to the invention are also described.

### Brief Description of the Drawings

While the specification concludes with claims particularly pointing out and distinctly claiming the present invention, it is believed the present invention will be better understood from the following description in which:
Figure 1 is a plan view photograph of a macroscopically expanded, plain surfaced three-dimensional plastic web of the type generally disclosed in the aforementioned patent application of Radel et al., said web being shown approximately two times actual size;
Figure 2 is a photograph generally similar to that of Figure 1, said web being shown approximately 18 times actual size;
Figure 3 is a photograph of the web shown in Figures 1 and 2 taken at an angle of approximately 55° with respect to the plane of said web, said web being shown approximately two times actual size;
Figure 4 is a photograph generally similar to that of Figure 3, said web being shown approximately 18 times actual size;
Figure 5 is a plan view photograph of a macroscopically expanded three-dimensional plastic web of the type generally disclosed in Figures 1-4, but incorporating a pattern of irregularly sized and irregularly spaced nubbles of the type disclosed in the aforementioned patent application of Sorenson on its visible surface, said web being shown approximately two times actual size;
Figure 6 is a photograph generally similar to that of Figure 5, said web being shown approximately 18 times actual size;
Figure 7 is a photograph of the web generally shown in Figures 5 and 6 taken at an angle of approximately 55° with respect to the plane of said web, said web being shown approximately two times actual size;
Figure 8 is a photograph generally similar to that of Figure 7, said web being shown approximately 18 times actual size;
Figure 9 is a plan view photograph of a preferred embodiment of a macroscopically expanded three-dimensional web of the present invention, said web being shown approximately two times actual size;
Figure 10 is a photograph generally similar to that of Figure 9, said web being shown approximately 18 times actual size;
Figure 11 is a photograph of the web generally shown in Figures 9 and 10 taken at an angle of approximately 55° with respect to the plane of said web, said web being shown approximately two times actual size;
Figure 12 is a photograph generally similar to that of Figure 11, said web being shown approximately 18 times actual size;
Figure 13 is a plan view photograph of another preferred embodiment of a macroscopically expanded three-dimensional plastic web of the present invention, said web being shown approximately two times actual size;
Figure 14 is a photograph generally similar to that of Figure 13, said web being shown approximately 18 times actual size;
Figure 15 is a photograph of the web generally shown in Figures 13 and 14 taken at an angle of approximately 55° with respect to the plane of said web, said web being shown approximately two times actual size;
Figure 16 is a photograph generally similar to that illustrated in Figure 15, said web being shown approximately 18 times actual size;
Figure 17 is a simplified schematic illustration of a preferred method and apparatus used to produce a macroscopically expanded, three-dimensional plastic web of the present invention;
Figure 18 is an enlarged end view of the debossing and perforating cylinder shown in Figure 17;
Figure 19 is an enlarged perspective view of the debossing and perforating cylinder shown in Figures 17 and 18;
Figure 20 is an enlarged, partially exploded segment of a preferred laminate film forming structure of the present invention (shown prior to rolling and seaming), said structure being particularly suitable for use in conjunction with one-sided forming processes of the type generally disclosed herein;
Figure 21 is a simplified schematic illustration of a preferred two-sided film forming process of the present invention;
Figure 22 is a simplified schematic illustration of a preferred one-sided film forming process of the present invention wherein a resinous melt is extruded directly onto the surface of a debossing and perforating cylinder having a microscopic pattern of surface aberrations thereon; and
Figure 23 is a simplified schematic illustration of a preferred two-sided film forming process of the present invention wherein a resinous melt is extruded directly onto the surface of a debossing and perforating cylinder and the opposite surface of said melt is thereafter contacted by a suction roll to impart a microscopic pattern of surface aberrations thereto.

### Detailed Description of the Invention

It is generally known in the art that the appearance attributes of any object are related to the ways in which the object modifies the light that strikes it. The light can be modified spatially resulting in diffuse and specular reflection and/or diffuse and specular transmission. The light can also be modified spectrally (in color). As is pointed out, beginning at page 26, of a prior art reference entitled "The Measurements of Appearance" by Richard F. Hunter, John Wiley & Sons, New York, 1975, four major things can happen to light when it encounters an object: (1) specular reflection at the skin of the object (associated with gloss); (2) scattering within the material (associated with diffuse reflection and sometimes with diffuse transmission); (3) absorption within the material (largely responsible for color); and (4) specular transmission directly through the object, if it is more or less transparent (associated with clarity).

The present invention is concerned primarily with the first two phenomena, particularly the aspects of specular reflection and diffuse reflection. As utilized herein, specular reflection is considered to occur when a beam of light is reflected from a surface such that the angle of reflection is equal to the angle of incidence, and the component of its direction which is parallel to the plane of said surface remains unchanged. Diffuse reflection, on the other hand, comprises any reflection which is non-specular. An example of a surface exhibiting only specular reflection would be a perfect mirror. It will therefore be appreciated that nearly any real surface will exhibit both specular and diffuse reflection. The aforementioned reference of Hunter points out, beginning at page 26, that it is specular reflection which is responsible for the visible glossy appearance of a surface. With normal nonmetallic materials, the specularly reflected light is not changed in color. Accordingly, the highlights of the object being observed appear white, the color of the light source.

Conversely, if the surface of the object in question exhibits primarily diffuse reflection with respect to an incident beam of light, the surface tends to appear more matt. In general, the more diffuse the reflection from an opaque surface, the more mat will be the appearance. However, it has been observed that there does not appear to be any clearly defined point at which a surface ceases being perceived as glossy and suddenly appears to be matt. In fact, there seems to be a gradual transition in perception from glossy to mat with various stages in between typically being described with words like "satiny".

It is also generally known in the art that the amount of light reflected or transmitted by an object can vary as the direction of view is changed. Curves showing change of amount of reflectance or transmittance with change of angle of view are normally called goniophotometric curves. Such curves identify the properties of specimens responsible for gloss, haze, luster, and other geometric attributes. Basically, a goniophotometric curve is produced by fixing the light source at a specific angle and measuring the light reflected at different angles of view. A goniophotometric curve is typically plotted on a two-dimensional X-Y coordinate system by designating the reflectance factor as the Y-axis and the angle of reflection as the X-axis. The reflectance factor scale is typically plotted logarithmically. If, for example, such a curve showing the amount of light reflected in various directions with the specimen illuminated at an angle of 45° is plotted, a surface which is high in specular reflection will exhibit a very high peak value at an angle of -45° (i.e., the point at which the angle of incidence with the web and the angle of reflection from the web are equal) and a minimal area under the curve thus formed, while a surface which is very mat will generally exhibit little evidence of a peak and a rather large area under the curve. It will of course be recognized by those skilled in the art that if the reflectance factor scale were plotted linearly rather than logarithmically, then the total area under each curve would be similar. For example, a logarithmically plotted goniophotometric curve for a perfect mirror would be a substantially vertical spike at -45°, while the curve for a perfectly mat surface would be a substantially horizontal line extending generally parallel to the X-axis at a lesser amplitude than said vertical spike.

According to the teachings of Hunter, a variety of established methods of gloss measurement have been developed over the years due to the necessity of measuring different aspects of reflection in order to duplicate, as far as possible, the different visual gloss grading procedures which are possible with the human eye. A more complete description of these generally accepted methods may be found in the aforementioned reference of Hunter, beginning at page 71.

Hunter expressly recognizes the relatively limited abilities of instruments typically employed when compared to the human eye. He suggests, at page 76, that the human eye has a higher resolving power than do most reflectance measuring instruments. According to Hunter, the normal human eye can see two lines as being separate when they are only 0.01° apart, while photometric measurements of reflectance are typically limited to receptor field angles about 100 times as large. Furthermore, the requirement for flat test surfaces for narrow-angle measurements of high gloss is very important when dealing with quantitative instrument measurements because an instrument cannot differentiate between a low reflectance rating due to poor image-reflecting quality of the specimen and a low reflectance rating caused by surface curvature that has directed the specularly reflected light beam improperly. The eye, however, can readily distinguish between these effects, since specimen nonflatness distorts, but does not destroy, the visual images reflected in its surface.

Accordingly, attempts to quantify gloss utilizing prior art measuring techniques and to meaning-fully correlate such quantitative measurements with a human observer's visual perception have not been completely successful. This is believed due to the fact that such prior art techniques take into consideration only one of the criteria which must be met to provide a surface which is perceived by the human eye as substantially non-glossy.

It has, in reducing the present invention to practice, been confirmed that the ability of a surface to exhibit primarily diffuse reflection is a necessary condition to minimize visually perceived gloss. However, the ability of a surface to exhibit diffuse reflection is not, in and of itself, a sufficient criteria to create the visual perception of a mat surface. A second criteria which must also be met is that the diffusing pattern must be sufficiently fine that it is nondiscernible to the naked eye of the observer under normal circumstances. If the pattern is discernible by the naked eye, then the surface will be visually perceived as a basically glossy surface with disruptions on it.

Experience with macroscopically expanded three-dimensional plastic webs of European Application Nos. 80200156.0 and 80200365.7 has demonstrated that gloss is visually perceived either: (a) when there is no pattern of surface aberrations to diffusely reflect the incident light; or (b) when the pattern of surface aberrations is discernible to the naked eye. Conversely, experience in reducing the present invention to practice has demonstrated that when the pattern of surface aberrations causes the surface to exhibit primarily diffuse rather than specular reflection and the pattern of surface aberrations is of a scale fine enough that it is not discernible to the naked eye under normal use conditions, then the surface is visually perceived as being mat.

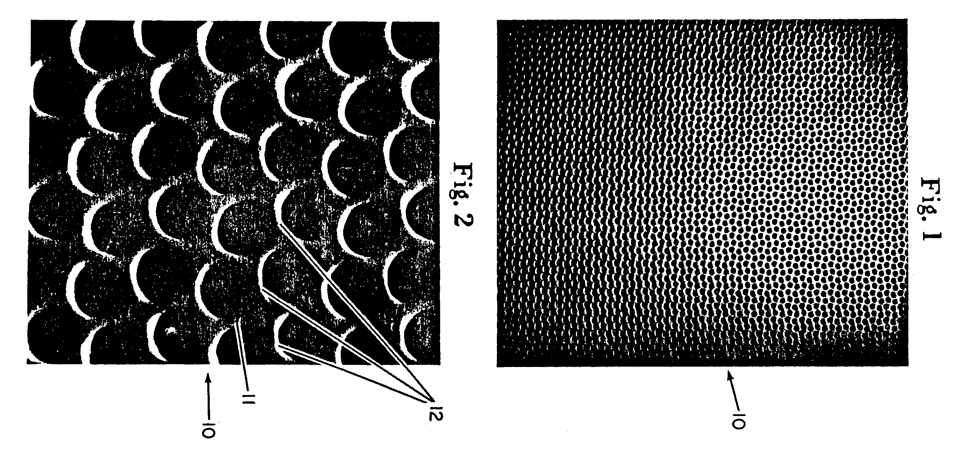

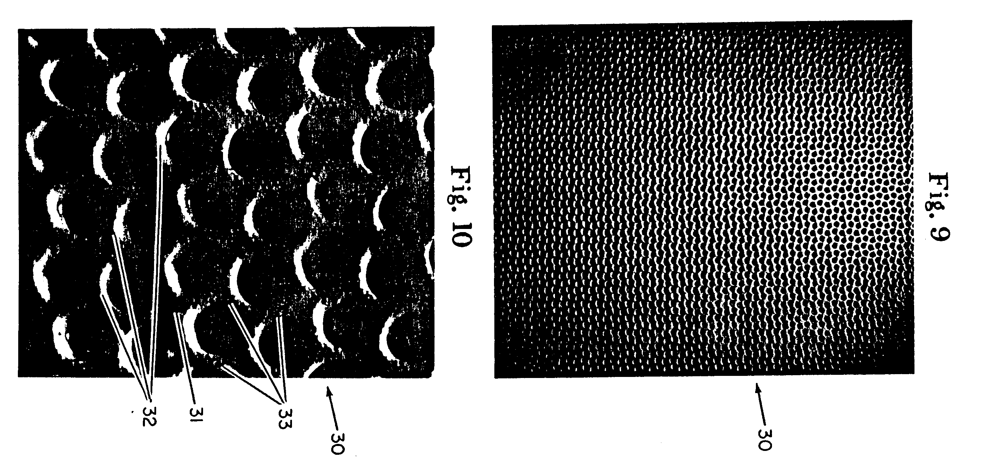

Exemplary embodiments of macroscopically expanded three-dimensional plastic webs of the type disclosed in the aforementioned European patent applications are disclosed in Figures 1-4 and 5-8, respectively, while exemplary embodiments of macroscopically expanded three-dimensional plastic webs of the present invention are disclosed in Figures 9 through 16.

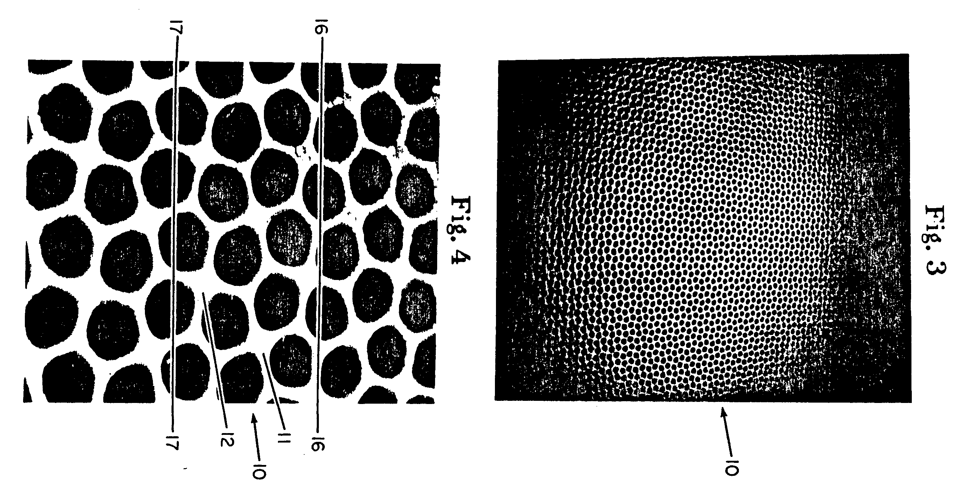

Figures 1, 2, 5, 6, 9, 10, 13 and 14 are all plan view photographs taken with the incident light forming an angle of approximately 20° with the plane of the webs. Thus, for the plan view photographs described above the angle formed between the camera lens and the incident light is approximately 70°. As pointed out earlier herein, maximum specular reflection would normally occur when the angle of light reflectance is equal to the angle of light incidence. Accordingly, Figures 3, 4, 7, 8, 11, 12, 15 and 16 are photographs taken when the sample has been repositioned to make the camera coincide with the angle of reflectance of the incident light to illustrate the condition existing at the point of maximum specular reflection. The incident light angle for these photographs was 55° with respect to the plane of the web, and the angle of reflectance, i.e., the angle at which the camera was positioned, was -55° with respect to the plane of the web.

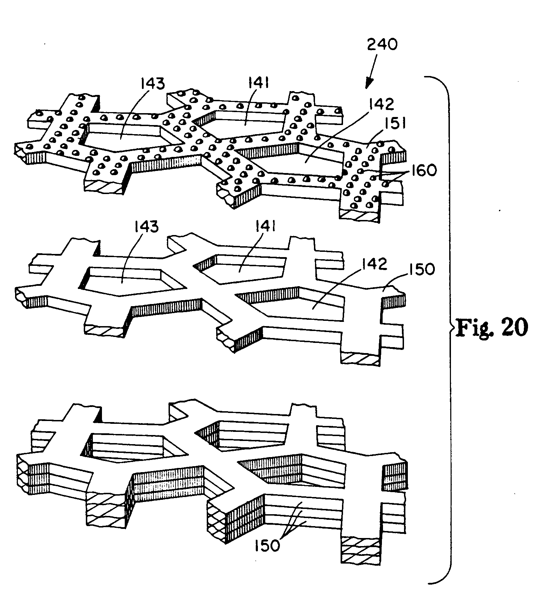

The webs disclosed in Figures 1-16 all exhibit a similar macroscopically expanded three-dimensional pattern. The forming structure utilized was generally similar to that disclosed in Figure 20 and was comprised of a stack of identical photoetched laminae, each having a multiplicity of irregularly shaped pentagonal apertures therein. The maximum dimension of said pentagonally shaped apertures was approximately 35 mils (i.e., 0.875 mm). Each lamina exhibited a thickness of approximately 10 mils (i.e., 0.254 mm). Five such lamina were superposed with their pentagonally shaped apertures in vertical alignment to provide a forming structure exhibiting a multiplicity of pentagonally shaped capillary networks having sidewalls oriented substantially perpendicular to the plane of plastic webs processed thereon. The laminae were secured to one another to form an integral structure generally in accordance with the teachings of the European patent application No. 80200156.0.

The uppermost lamina of the forming structure utilized to produce the macroscopically expanded three-dimensional plastic web 10 disclosed in Figures 1-4 was left untreated, i.e., no pattern of surface aberrations was provided on its uppermost surface.

The web 10 is shown about two times actual size in Figures 1 and 3 to lend some perspective to the fineness of the three-dimensional pattern, and about 18 times actual size in Figures 2 and 4 to clarify the gloss phenomenon. Due to the limitations inherent in photographic techniques, the gloss phenomenon perceived by the human eye when examining the actual samples is not readily apparent from an examination of the photographs taken at the lower scale magnification.

While the highly enlarged plan view photograph of Figure 2 shows only isolated gloss highlights 12 on the web's visible surface 11, the photograph of Figure 4, which coincides with the angle of reflectance for the incident light exhibits a substantially continuous gloss highlight on the web's visible surface. In this regard it should be noted that only those portions of Figure 4 located intermediate view lines 16-16 and 17-17 are in proper focus due to the angular orientation of the web 10 with respect to the camera. It is this specularly reflected light which is visually perceived as gloss by the observer when the web 10 is subjected to normal use.

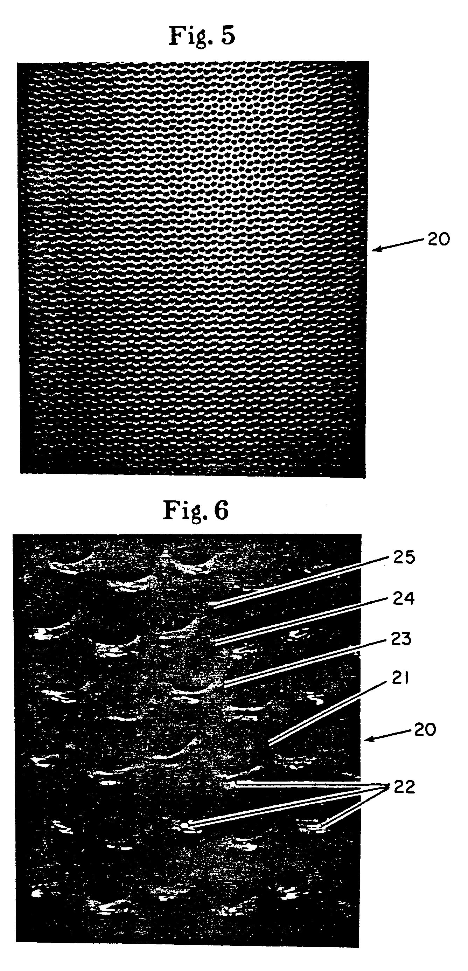

The forming structure utilized to produce the macroscopically expanded three-dimensional plastic web 20 disclosed in Figures 5-8 was treated with an epoxy grit mixture generally in accordance with the teachings of U.S. Patent No. 4,259,286 and European Patent Application No. 80200365.7. In particular, a mixture comprised of 70 percent No. 400 Grit and 30 percent No. 325 Grit was mixed with a powdered epoxy in a ratio of approximately 3:2 (grit : epoxy) and sprayed as a powder onto the uppermost surface of the electrostatically charged forming structure. Once deposited on the surface of the forming structure the mixture of grit and epoxy were baked generally in accordance with the teachings of U.S. Patent No. 4,259,286 to secure the grit permanently in place.

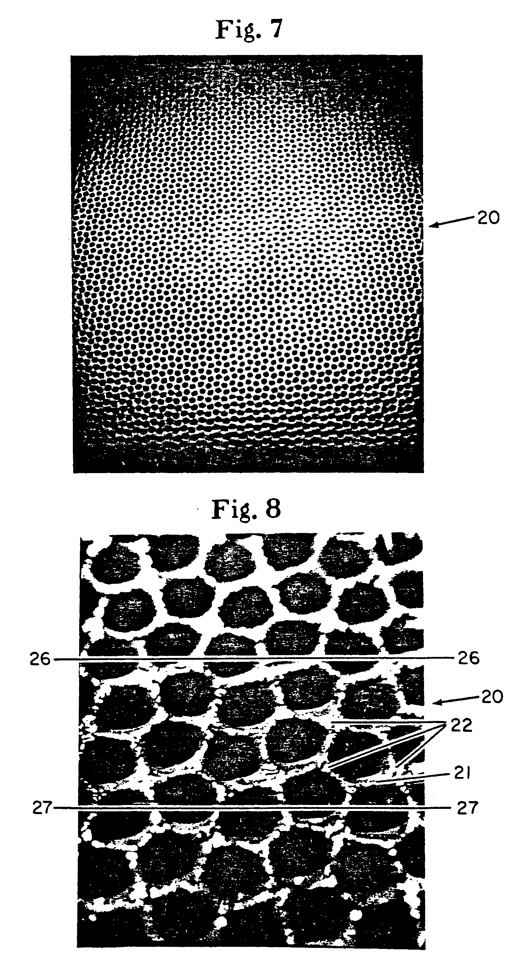

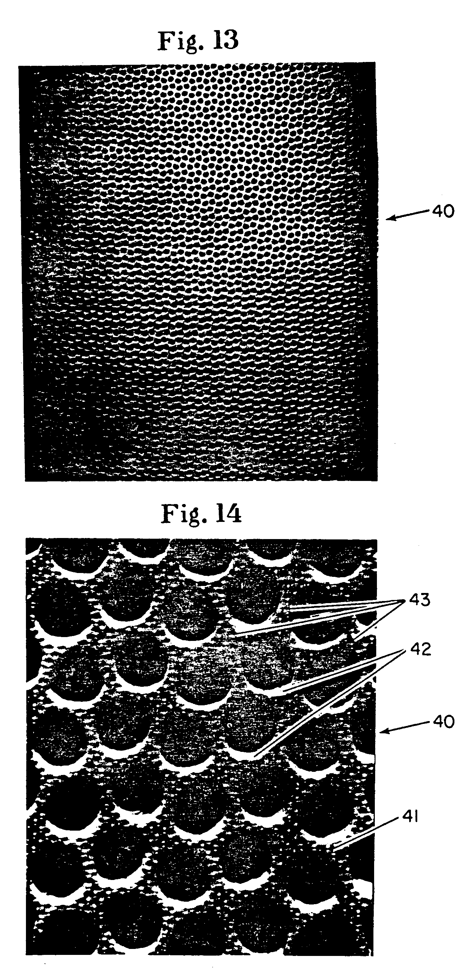

As can be seen in Figure 6, the visible surface 21 of web 20 is provided with a multiplicity of irregularly sized and irregularly spaced nubbles, e.g., nubbles 23, 24, 25. As with the web 10, shown in Figures 1-4, the plan view photograph of Figure 6 shows only isolated gloss highlights 22 on the web's visible surface 21, while the photograph of Figure 8, which coincides with the angle of reflectance of the incident light, exhibits much more extensive gloss highlights on the web's visible surface. Nonetheless, there is generally less visually perceived gloss under normal use conditions with webs of the type disclosed in Figures 5-8 than with webs of the type disclosed in Figures 14 due to the presence of the nubbles, e.g., nubbles 23, 24, 25. As with Figure 4, only those portions of Figure 8 located intermediate view lines 26-26 and 27-27 are in proper focus due to the angular orientation of the web 20 with respect to the camera.

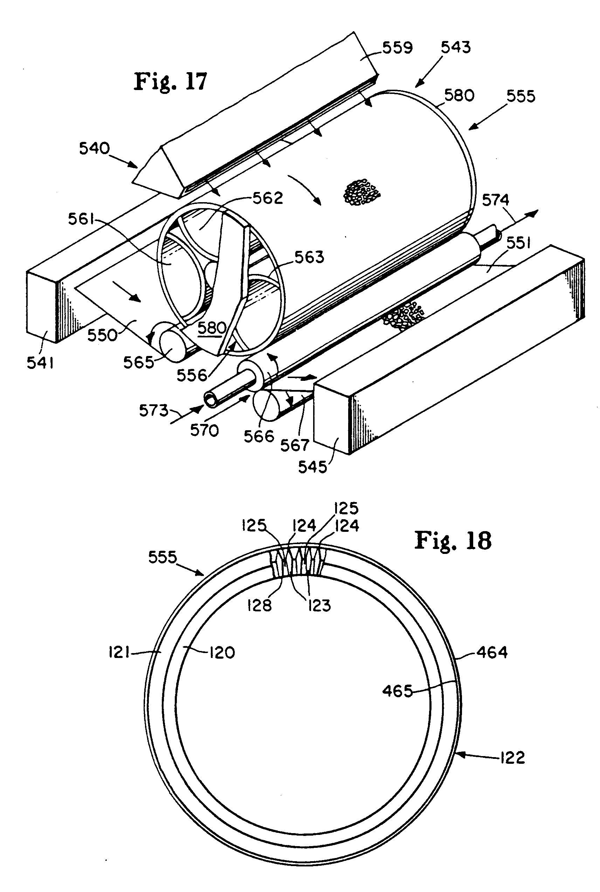

The web 30 shown in Figures 9-12 is a preferred embodiment of the present invention produced utilizing a one-sided forming process of the type generally disclosed in Figure 17. The uppermost lamina of the forming structure utilized to produce the macroscopically expanded three-dimensional plastic web 30 disclosed in Figures 9-12 was provided with a microscopic pattern of regularly spaced surface aberrations generally similar to surface aberrations 160 disclosed in Figure 20. The aberrations, which comprised protuberances projecting generally outwardly from the surface of the web, were circular in cross-section, as measured in a plane oriented perpendicular to their amplitude, i.e., a plane parallel to the surface of said web. They had an overall cross-sectional diameter of approximately four mils (i.e., 0.1 mm) and were arranged in a hexagonally close packed pattern with a four mil (i.e., 0.1 mm) center-to-center spacing between adjacent surface aberrations. The average amplitude of the surface aberrations was approximately 0.3 mils (i.e., 0.0075 mm). The surface aberrations were provided in the uppermost lamina of the forming structure after the pentagonally shaped apertures had been provided therein by a secondary photoetching treatment of the type generally disclosed in the aforementioned patent application of Radel et al.

The amplitude of the surface aberrations referred to throughout the present specification and claims is preferably measured using a high powered microscope which enables the operator to measure distance perpendicular to the plane of the sample as a function of focus. In particular, this method employs a top lighted metallurgical microscope with a calibrated focusing wheel. While the degree of magnification is typically adjustable on these units, a magnification of 400× has been found to provide a good compromise between depth of focus and viewing area.

Samples of the web to be measured are preferably prepared by cutting a rectangular section approximately 12 mm by 36 mm from the web. These samples are then mounted on individual microscope slides using double-sided tape with their visible surface upwardly oriented. The visible surface of each sample is preferably darkened with a black felt tip marker. Since webs of the present invention may be completely transparent, completely opaque, or anywhere between these two extremes, the ink applied by the felt tip marker permits accurate amplitude measurement regardless of the degree of transparency or opacity of the particular web being measured. Darkening in the aforementioned manner with a felt tip marker provides improved contrast between the uppermost surface of the aberration and the plane of origination adjacent the aberration to be measured. Due to the tendency of the ink to run from the uppermost surface of the aberration, the uppermost surface will normally appear somewhat lighter under the microscope, while the plane of origination, which is generally perceived as a valley, will appear generally darker in color. A Sanford Sharpie No. 3000 black felt tip marker, such as is available from Sanford Corporation of Bellwood, Illinois, may be employed for the aforementioned purpose.

Once the web sample has been prepared, the following procedure is preferably carried out:
1. Place the prepared sample on the microscope with the visible surface of the sample oriented toward the lens.
2. Focus on the top of a particular surface aberration and then on the plane of origination adjacent said aberration to identify distinguishing features to be used in determining focus. As mentioned earlier herein, the uppermost surface of the aberration will normally appear somewhat lighter in color, while the plane of origination adjacent the aberration will appear generally darker in color.
3. Adjust the focus of the microscope until it is above the plane of the uppermost surface of the particular surface aberration to be measured.
4. Turn the focus wheel in one direction only until the uppermost surface of the particular surface aberration just comes into focus. Stop turning at this point and record the reading from the focus wheel.
5. Continue turning in the same direction until the plane of origination adjacent said aberration just comes into focus. Stop turning the focus wheel and again record the reading from the focus wheel.
6. The difference between these two readings on the focus wheel represents the amplitude of the particular surface aberration from its plane of origination. Since the focus wheel is calibrated the distance of travel can readily be converted into mils.

The aforementioned procedure can also be used in situations wherein the surface aberration comprises a depression rather than a protuberance, the readings preferably being taken first. in the plane of origination and then at the point of maximum amplitude.

When using the aforementioned measurement method, it is preferred that the focusing be accomplished by turning the focus wheel in one direction only. This eliminates any end play in the focus wheel mechanism which could be significant when compared to the values being measured. It should also be noted that the leading edge of the focus field is preferably used when the measurements described above are recorded in order to minimize the effect of a finite, i.e., greater than zero, depth of field. This is accomplished by stopping and recording the reading on the focus wheel when the surface in question just comes into focus, rather than at the point corresponding to the middle of the focus field or the point at which the trailing image is about to go out of focus. When this is done for both the first and the second readings, the effect of finite depth of field is substantially eliminated from the amplitude measurement.

While nearly any microscope with top light capability, magnification power of 400× (or greater) and a calibrated focusing wheel may be utilized to perform the above measurements, particularly good results have been obtained utilizing an ausJena Neophot 21 metallurgical microscope set at 400×. This particular equipment is manufactured in Jena, East Germany and is distributed in the U.S.A. by the Leco Corporation of Warrendale, Pennsylvania. If desired, the unit may also be equipped with a television camera and video monitor for easier viewing.

To ensure that the amplitude determination made utilizing the foregoing technique is representative for the entire surface of the web sample, each of the 12 mm by 36 mm web samples is preferably measured in at least 16 individual locations selected at random. The average amplitude for any given sample is computed by calculating the arithmetic average of all measurements taken from a given web sample. Thus, the average amplitudes reported throughout the present specification and claims represent the arithmetic average for the sample, as determined by the foregoing technique.

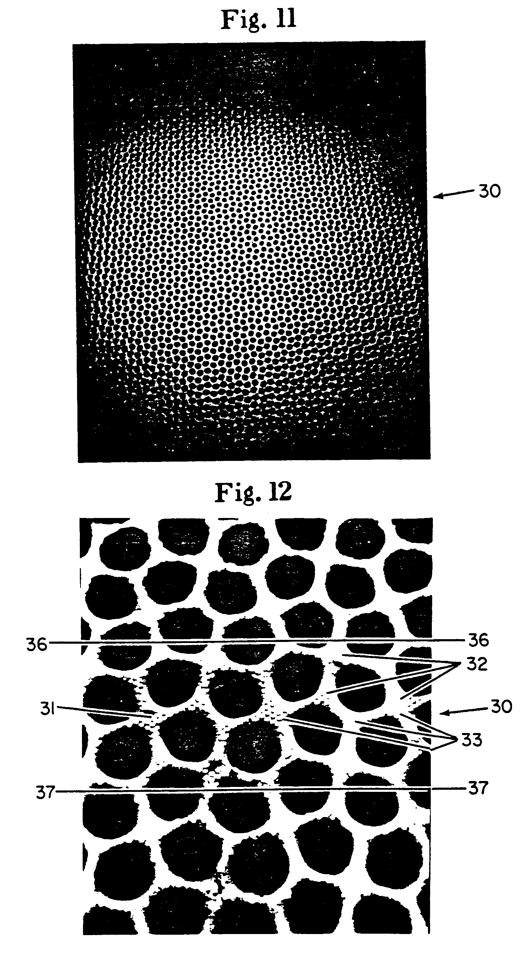

As can be seen in Figure 12. the visible surface 31 of the web 30 formed on the structure described earlier herein is provided with a multiplicity of regularly sized and spaced surface aberrations 33 corresponding to the surface aberrations on the forming structure. As with the webs 10 and 20 shown in Figures 1-4 and 5-8, respectively, the plan view photograph of Figure 10 shows a number of isolated glass highlights 32 on the web's visible surface 31. However, unlike the photographs of Figures 4 and 8 the photograph of Figure 12, which coincides with the angle of reflectance of the incident light, exhibits only slightly more extensive gloss highlights on the web's visible surface than the plan view photograph of Figure 10. Accordingly, when the web is subjected to normal use, the microscopic pattern of surface aberrations serves to diffusely reflect a substantial portion of the light incident upon the web's visible surface. Furthermore, because the pattern of surface aberrations causing diffuse reflection of the incident light cannot be discerned when the perpendicular distance between the observer's eye and the plane of the web is 30 cm or greater, the web is perceived as substantially non-glossy. Finally, because the average amplitude of the surface aberrations 33 is 0.3 mils (i.e., 0.0075 mm), the tactile impression exhibited by the web is generally perceived to be more cloth-like or fiber-like than for webs of the type illustrated in Figures 1-8.

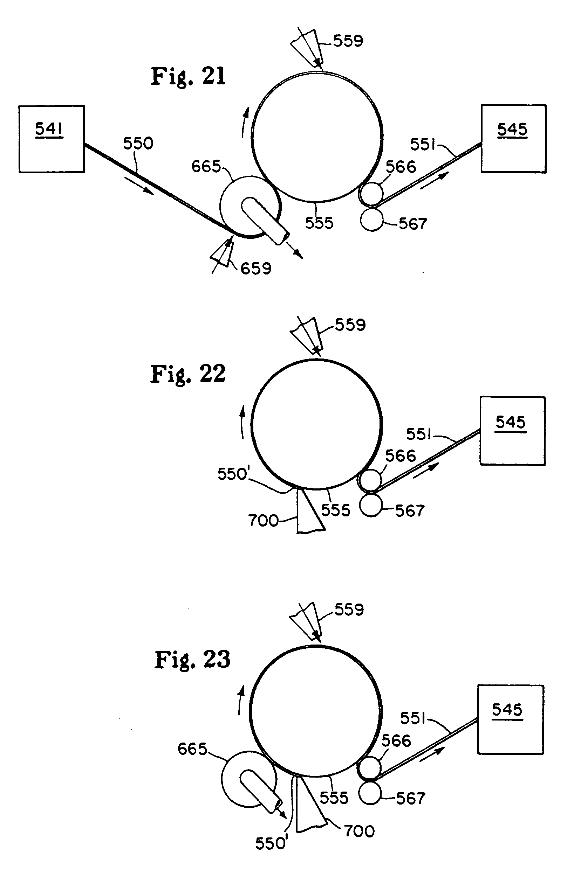

The macroscopically expanded three-dimensional plastic web 40 generally disclosed in Figures 13-16 is another preferred embodiment of the present invention produced utilizing a two-sided forming process of the type generally shown in Figure 21. The web 40 was processed by subjecting its visible surface 41 to suction while the web was supported at an elevated temperature on a woven wire mesh forming structure. The wire mesh was woven from filaments having a diameter of 1.6 mils (i.e., 0.04 mm) in a square weave pattern and exhibited a mesh count of approximately 96 filaments per lineal cm by 96 filaments per lineal cm. The web was thereafter macroscopically expanded on a plain-surfaced three-dimensional forming structure. As a result, the visible surface of the web 40 exhibits a microscopic pattern of regularly spaced surface aberrations 43 which, in this case, comprise depressions corresponding to the knuckles of the aforementioned woven wire mesh forming structure.

As can be seen in the greatly enlarged plan view photograph of Figure 14, only isolated gloss highlights 42 are observable on the web's visible surface 41.

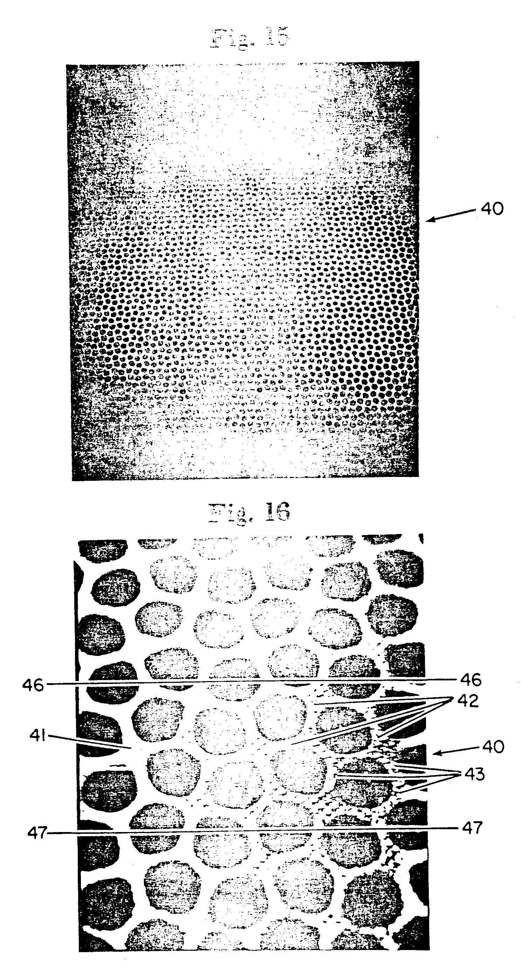

The reduction in gloss is even more apparent from the photograph of Figure 16, which coincides with the angle of reflectance of the incident light. Figure 16 exhibits only a limited number of very small gloss highlights 42 due to the fine scale pattern of surface aberrations 43 which serve to diffusely reflect the incident light. When the web 40 is subjected to normal use, the microscopic pattern of regularly spaced surface aberrations 43 is not discernible when the perpendicular distance between the observer's eye and the plane of the web is about 12 inches or greater. This lack of visual discernibility can readily be verified from an inspection of Figures 13 and 15 which are shown approximately two times actual size.

Because of the average amplitude of the surface aberrations 43 on web 40 is 0.3 mils (i.e., 0.0075 mm) the web 40 exhibits a tactile impression generally similar to that of the web 30 disclosed in Figures 9-12.

From the foregoing it is apparent that the present invention has identified two key criteria which must be simultaneously satisfied for a surface which is normally glossy, e.g., an opaque plastic web, to appear mat under normal use circumstances. First, the surface must diffusely reflect rather than specularly reflect a substantial portion of the light incident upon it, and second, the regularly spaced, microscopic pattern of surface aberrations utilized to diffuse the light, which must be present throughout the visible surface of the web, must not be discernible to the naked eye when the web or the object embodying the web is subjected to normal use.

Applying these criteria to substantially opaque macroscopically expanded three-dimensional plastic webs which may be employed as alternatives for cloth and fibrous structures which contact the wearer's skin, as well as for new product applications, it has been observed that unless a person holding an object in his or her hands is attempting to examine the nature of the object in detail, the hands are normally maintained a distance of at least 30 cm or more away from the eyes. Based upon this observation, it has been determined that a macroscopically expanded three-dimensional plastic web can be provided with a substantially non-glossy visible surface when the visible surface of the web is made to exhibit a regularly spaced, microscopic pattern of surface aberrations which are not discernible to the normal naked eye when the perpendicular distance between the observer's eye and the plane of the web is 30 cm or greater.

The regularity of pattern and spacing, which is not embodied in the macroscopically expanded three-dimensional plastic web 20 disclosed in Figures 5-8, is necessary to ensure that all portions of the web's visible surface exhibit the desired characteristics. If substantially less than the entire visible surface of the web exhibits the desired characteristics, gloss will be perceived in those areas which fail to comply, and the desired cloth-like or fiber-like impression may be lost with respect to the entire web.

It has further been determined in reducing the present invention to practice that in order to provide primarily diffuse rather than specular reflection, each of the aforementioned surface aberrations must be substantially free of planar areas which are large enough to inscribe a four mil (i.e., 0.10 mm) diameter circle, and must be so spaced relative to all adjacent surface aberrations that the maximum diameter of any circle which can be inscribed on any planar surface intermediate said surface aberration and said adjacent surface aberrations is less than about four mils (i.e., 0.10 mm). These criteria are not satisfied by either of the web embodiments disclosed in Figures 1-4 or 5-8.

A check for the presence of planar areas which are large enough to inscribe a four mil diameter circle on the surface of a particular web sample may be made by orienting the surface or portion thereof to be examined substantially perpendicular to the viewing angle of a top lighted metallurgical microscope of the type described earlier herein in connection with measuring the amplitude of the surface aberrations. Samples of the web to be measured are prepared in a manner similar to that described in connection with the aforementioned amplitude measurements. The visible surface of each sample is preferably darkened with a black felt tip marker to permit accurate measurement regardless of the degree of transparency or opacity of the web.

Using a high magnification, preferably 1000×, a plane may be defined as any area which appears simultaneously in focus. The previously described four mil diameter circle can be inscribed in the particular planar area being examined if the boundary of the four mil diameter circle and all of the area contained therein appear to be simultaneously in focus. This examination is most easily facilitated by coupling a television camera and video monitor with a correspondingly enlarged four mil diameter circle overlaid on the screen of the video monitor to the microscope. Because the depth of field for focusing is shallow with a magnification of 1000×, it can be seen that movement of ±0.02 mils (±0.0005 mm) from a focused position is more than sufficient to render total obscurity of all details being examined due to blurring. Since naturally occurring surface imperfections on smooth web samples which do not exhibit surface aberrations of the type herein disclosed generally fall within the foregoing range, this level of accuracy is considered adequate for determining whether or not a four mil diameter circle may be inscribed in the area being examined.

It has been further learned in practicing the present invention to advantage that the aforementioned surface aberrations may comprise protuberances projecting generally outwardly from the surface of the web or depressions projecting generally inwardly from the surface of the web.

Unexpectedly, it has also been learned that a more cloth-like or fiber-like tactile impression can be obtained in macroscopically expanded three-dimensional webs of the present invention when the surface aberrations described earlier herein have an average amplitude of at least 0.2 mils (i.e., 0.005 mm), most preferably at least 0.3 mils (i.e., 0.0075 mm), as measured perpendicularly from the top of the protuberance or the bottom of the depression, as the case may be, to the plane in which said surface aberration originates.

For persons having normal vision, it has been determined that in order to maintain non-discernibility of the pattern of surface aberrations when the perpendicular distance between the viewer's eye and the plane of the web is 30 cm, the maximum dimension of any of said surface aberrations should be less than 6 mils (i.e., 0.15 mm), as measured in a plane substantially perpendicular the amplitude of said surface aberrations, i.e., a plane substantially parallel to the surface of said web.

The macroscopically expanded three-dimensional plastic webs disclosed in Figures 9-12 and 13-16, which are preferred embodiments of the present invention, satisfy the aforementioned criteria for a substantially non-glossy visible surface as well as the aforementioned criteria for a more cloth-like or fiber-like tactile impression.

A particularly preferred continuous forming process which may be employed in practicing the present invention on webs comprised of plastic film and having a substantially uniform planar thickness of about 0.05 mm (two mils) or less, as measured prior to any macroscopic expansion thereof, is schematically illustrated in Figure 17. This process is generally described in commonly assigned U.S. Patent 4,151,240 issued to Malcolm B. Lucas and Robert H. Van Coney on April 24, 1979.

The particularly preferred apparatus 540 shown in Figure 17 includes constant tension film supply means 541, debossing and perforating means 543, and constant tension film forwarding and winding means 545. The frame, bearings, supports and the like which must necessarily be provided with respect to the functional members of apparatus 540 are not shown or described in detail in order to simplify and more clearly depict and disclose the present invention, it being understood that such details would be obvious to persons of ordinary skill in the art of designing plastic film converting machinery.

Briefly, apparatus 540, Figure 17, comprises means for continuously converting a planar ribbon of thermoplastic film 550 into a macroscopically expanded three-dimensional film 551 by directing hot air jets against one surface of the film while applying vacuum adjacent the opposite surface of the film, and while maintaining sufficient control of the film to substantially obviate wrinkling and/or macroscopically distending the film. Thus, as will be more fully described hereinafter, the apparatus 540 comprises means for maintaining constant machine direction tension in the film both upstream and downstream of a zone where the temperature is greater than the thermoplastic temperature of the film, but in which zone there is substantially zero machine direction and cross-machine direction tension tending to macroscopically distend the film. The aforementioned upstream and downstream tension is required to control and smooth the running ribbon of thermoplastic film. The zero tension zone results from the film in the zone being at a sufficiently high temperature to enable debossing and, if desired, perforating or aperturing it through the use of heat and vacuum. The perforations shown in Figure 17 are greatly enlarged to enable visually perceiving the nature of the difference between the imperforate planar film 550 and the resulting macroscopically expanded three-dimensional film 551, as more fully described hereinafter.

As can be seen in Figure 17, the debossing and perforating means 543 includes a rotatably mounted debossing/perforating cylinder 555 having closed ends 580, a nonrotating triplex vacuum manifold assembly 556 and hot air jet means 559. The triplex vacuum manifold assembly 556 comprises three manifolds designated 561, 562 and 563. Also shown in Figure 17 is a freely rotatable lead-on idler roll 565, a power rotated lead-off/chill roll 566, and a soft-faced (e.g., low density neoprene) roll 567 which is driven with the chill roll.

Briefly, by providing means (not shown) for independently controlling the degree of vacuum in the three vacuum manifolds, a thermoplastic ribbon of film running circumferentially about a portion of debossing/perforating cylinder 555 is sequentially subjected to a first level of vacuum by manifold 561, a second level of vacuum by manifold 562, and a third level of vacuum by manifold 563. As will be described more fully hereinafter, vacuum applied to the film by manifold 561 enables maintaining upstream tension in the film, vacuum applied by manifold 562 enables three-dimensionally debossing and perforating the film when hot air is directed radially inwardly against the film, and vacuum applied by manifold 563 cools the film to below its thermoplastic temperature and enables establishing downstream tension therein. If desired, the film contacting surface of the debossing/perforating cylinder 555 may be preheated prior to reaching vacuum manifold 562 by means well known in the art (and therefore not shown) to facilitate better conformance of plastic films comprised of flow-resistant polymers to the forming structure during the debossing and perforating operation. The nip 570 intermediate chill roll 566 and the soft-faced roll 567 is only nominally loaded to avoid ironing out the three-dimensional debossments which are formed in the film in the aforementioned manner. However, even nominal pressure in nip 570 helps the vacuum applied by manifold 563 to isolate downstream tension (i.e., roll winding tension) from the debossing/perforating portion of the debossing/perforating cylinder 555, and enables the nip 570 to peel the three-dimensionally debossed and perforated film from the debossing/perforating cylinder 555. Moreover, while ambient air passing through the film as it is drawn by vacuum into manifold 563 will normally cool the film to below its thermoplastic temperature, the passage of coolant through the chill roll as indicated by arrows 573, 574 in Figure 17 will enable the apparatus to handle thicker films or to be operated at higher speeds.

To summarize, the first vacuum manifold 561, and the third vacuum manifold 563 located within the debossing/perforating cylinder 555 enable maintaining substantially constant upstream and downstream tension, respectively, in a running ribbon of film while the intermediate portion of the film adjacent the second vacuum manifold 562 within the debossing/perforating cylinder 555 is subjected to tension vitiating heat and vacuum to effect three-dimensional debossing and perforating of the film.

Referring again to Figure 17, the constant tension film supply means 541 and the constant tension film forwarding and winding means 545 may, if desired, be substantially identical to and function substantially identically to the corresponding portions of the apparatus shown and described in commonly assigned U.S. Patent 3,674,221 issued to Reimersma on July 4, 1972.

The debossing and perforating means 543 comprises the rotatably mounted debossing/perforating cylinder 555, means (not shown) for rotating the cylinder 555 at a controlled peripheral velocity, the non-rotating triplex vacuum manifold assembly 556 inside the debossing/perforating cylinder 555, means (not shown) for applying controlled levels of vacuum inside the three vacuum manifolds 561, 562, and 563 comprising the triplex manifold assembly 556, and hot air jet means 559.

The debossing/perforating cylinder 555 may be constructed by generally following the teachings of the aforementioned commonly assigned U.S. Patent of Malcolm B. Lucas and Robert H. Van Coney. However, the film-contacting surface of the tubular forming structure is provided with a fine scale pattern of surface aberrations corresponding to the pattern of surface aberrations desired in the visible surface of the resultant macroscopically expanded three-dimensional plastic film.

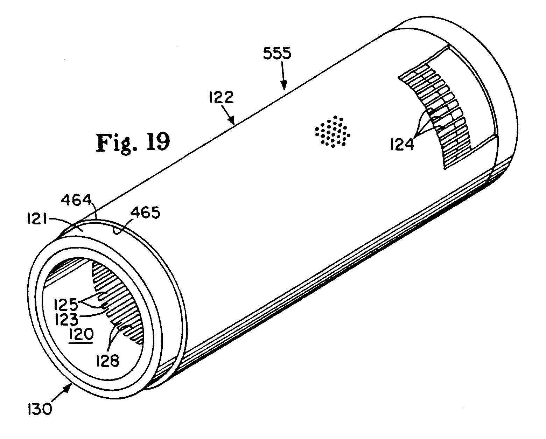

The debossing/perforating cylinder 555 shown in Figure 17 is illustrated in greater detail in Figures 18 and 19. The cylinder 555 comprises a cage 120, a support ring 121 and a relatively thin walled film-contacting tubular member 122. The cage 120 comprises a multiplicity of circumferentially spaced, longitudinally extending bars 123 which are tapered to relatively small, radially outwardly facing lands 124. The spaced bars 123 have vacuum communicating passageways 125 provided therebetween. The bars 123 also have radially inwardly facing lands 128 which corporately provide a cylindrical vacuum sealing surface against which the vacuum seals associated with the triplex vacuum manifold 556 are biased. Thus, as the debossing/perforating cylinder 555 rotates, its vacuum sealing surface slides over the seals (not shown) of the non-rotating triplex vacuum manifold assembly 556.

The end 130, Figure 19, of the debossing/perforating cylinder 555 disposed remotely from its driven end is open in order to provide easy insertion/removal of the triplex vacuum manifold assembly 556. Therefore, in order to rotatably support the open end 130 of cylinder 555, it is provided with a bearing-race support ring 121, as shown in Figures 18 and 19, which rides on bearings (not shown) which are appropriately secured to the apparatus frame (not shown).

Tubular member 122 is fluid pervious and may comprise a relatively thin laminate structure such as 240, a partially exploded, enlarged planar segment of which is shown in Figure 20, in contacting relation with the small lands 124 of the longitudinally extending support bars 123 of cage 120. The tubular member 122 is configured to deboss and perforate an extremely fine three-dimensional, apertured pattern into a relatively thin thermoplastic film such as low density polyethylene film, as will be described in greater detail hereinafter.

Only the outermost surface 464 of the tubular forming member 122 contacts the plastic webs brought in contact therewith. The innermost surface 465 of the tubular member contacts the lands 124 of support members 123 during the debossing/perforating operation.

The tubular member 122 shown in Figures 18 and 19 may be constructed generally in accordance with the teachings of European Patent Application No. 80200156.0.

The tubular member 122 may be constructed utilizing a stack of copper plated, photoetched metallic laminae exhibiting concentrically aligned patterns of apertures, said laminae being bonded to one another at contact points while subjected to heat and pressure. The resultant laminate structure is thereafter rolled into a tubular shape and its free edges are bonded to one another to form a continuous tubular forming structure generally in accordance with the teachings of the aforementioned European patent application.

Figure 20 is a simplified embodiment of a particular laminate structure 240 which could, if desired, be utilized to provide a surface suitable for debossing and perforating an initially imperforate, substantially planar plastic web to produce a fluid-pervious macroscopically expanded three-dimensional plastic web exhibiting a fine scale pattern of pentagonally shaped capillary networks, each of said networks having a substantially constant cross-section along its length. The laminate structure 240 (shown prior to rolling and seaming) is comprised of a stack of identically apertured laminae. With the exception of the pattern of surface aberrations 160 present on uppermost lamina 151, laminae 150 and 151 are identical to one another. Each lamina has a pattern of irregular pentagonally shaped openings or apertures, e.g., apertures 141, 142, 143, therein. In the illustrated embodiment, laminae 150 and 151 are so stacked that the pentagonally shaped apertures in each successive lamina coincide with one another.

Laminae 150 are preferably formed from planar metallic sheets by photoetching techniques well known in the art, as described in the aforementioned European Patent Application No. 80200156.0. The uppermost surface of lamina 151, which coincides with the visible surface of plastic webs contacting tubular member 122, is also preferably photoetched by techniques well known in the art to provide a regularly spaced, microscopic pattern of protuberances, hereinafter generally referred to as surface aberrations 160. This is preferably accomplished by applying a resist coating which corresponds to the desired microscopic pattern of surface aberrations to the top side of a planar photoetched lamina 150, and thereafter initiating a second photoetching process. The second photoetching process produces a lamina 151 having a microscopic pattern of surface aberrations 160 on the interconnected fiber-like elements defining the pentagonally shaped apertures, e.g., apertures 141, 142, 143.

In order to construct a forming structure suitable for producing substantially non-glossy macroscopically expanded three-dimensional plastic webs of the present invention, it is necessary that the microscopic pattern of surface aberrations 160 be sufficiently small that said pattern, when imparted to an opaque plastic web, is non-discernible when the perpendicular distance between the viewer's eye and the plane of said web is about 30 cm (12 inches) or greater, that each of said surface aberrations 160 be free of any planar areas which are large enough that when imparted to an opaque plastic web, the resultant surface aberration in said web will be free of any planar areas which are large enough to inscribe a four mil (i.e., 0.10 mm) diameter circle, and that each of said surface aberrations 160 be so spaced relative to all adjacent surface aberrations 160 that, when imparted to an opaque plastic web, the maximum diameter of any circle which can be inscribed on any planar surface intermediate said surface aberration on said web and said adjacent surface aberrations on said web is less than four mils (i.e., 0.10 mm).

Because the thickness of said web will influence the size and spacing of the surface aberrations on the forming structure, it should be noted that forming structures utilized to produce substantially non-glossy macroscopically expanded three-dimensional webs of the present invention will not necessarily satisfy the criteria which must be met by the resultant webs. Furthermore, even if the size and spacing criteria are met by the forming structure, said forming structures are typically comprised of metals or other non-plastic materials having vastly different reflectance characteristics than the opaque plastic webs processed thereon. Accordingly, visual discernibility of the pattern of surface aberrations or the visual perception of gloss on the forming structure does not necessarily mean that plastic webs processed thereon will also exhibit gloss.

As utilized herein, an "adjacent" surface aberration shall be defined as any surface aberration which can be included within a pair of unobstructed straight, radially extending lines originating at the geometric center of the aberration under consideration and making tangential contact with the aberration being tested for adjacency.

In order to provide a cloth-like or fiber-like tactile impression in the resultant macroscopically expanded three-dimensional plastic web, it has been found that the protuberances comprising surface aberrations 160 on lamina 151 should exhibit an amplitude, i.e., the perpendicular distance from the top of said surface aberration to the plane in which said aberration originates, that is sufficient to produce an average amplitude of at least 0.2 mils (i.e., 0.005 mm) in the resultant plastic web, and most preferably at least 0.3 mils (i.e., 0.0075 mm). In general, the greater the amplitude of the surface aberrations in the resultant plastic web, the more fiber-like said web will feel. In this regard it should also be noted that while the amplitude of said surface aberrations may vary considerably over the entire expanse of the resultant web, to optimize the fiber-like tactile impression of the web it is preferable that the amplitude of any particular surface aberration should not vary from the average value of the amplitude for all adjacent surface aberrations by more than ±20 percent, and most preferably not more than ±10 percent.

When the aforementioned criteria are met by the forming structure, the visible surface of webs which are macroscopically expanded so as to assume the three-dimensional pattern of said forming structure will appear non-glossy when the perpendicular distance between the viewer's eye and the plane of the web is 30 cm or greater. If desired, the pattern of surface aberrations 160 on lamina 151 can be designed so that both the lamina-contacting and the non-lamina-contacting surfaces of the web coinciding with uppermost lamina 151 will appear non-glossy. This is due to the fact that substantially all of the light incident upon the visible surface of the web will be diffused into a multiplicity of directions by said surface aberrations, yet the pattern causing said diffusion is non-discernible to the naked eye under normal use conditions.

Because the sidewalls of the capillary networks formed by the coinciding apertures, e.g., apertures 141, 142, 143, do not exhibit any surface aberrations, these surfaces would appear glossy if they formed a portion of the web's visible surface, as viewed from the non-lamina contacting side of the macroscopically expanded three-dimensional plastic web. However, since the sidewall surfaces are oriented substantially perpendicular to the web's visible surface in the disclosed embodiment, light emanating from the non-lamina contacting side of the web and incident upon the sidewalls will not, under most conditions, be specularly reflected at a viewer observing the non-lamina contacting surface of the web. Accordingly, the entire non-lamina contacting surface of the resultant web will exhibit a substantially non-glossy appearance.

Conversely, light emanating from the lamina-contacting side of the web and incident upon the sidewalls will be specularly reflected at a viewer observing the lamina contacting surface of the web. Accordingly, the viewer will generally perceive the lamina contacting surface of the web as being glossy, despite the fact that the portion of the web coinciding with lamina 151 might exhibit a non-glossy appearance.

From the foregoing, it is clear that in order to impart a non-glossy appearance to macroscopically expanded three-dimensional plastic webs of the present invention, it is necessary that substantially all portions of a web's visible surface, i.e., those portions of the web which are visible when viewed substantially perpendicularly to the plane of the web from the side of interest, must exhibit a pattern of surface aberrations which satisfy the criteria set forth earlier herein. Thus, for forming structures exhibiting straight sidewalls which are oriented substantially perpendicular to the plane of the web, only those surfaces visible from an overhead plan view of the forming structure (when said structure is in a planar condition) need be provided with said pattern of surface aberrations. However, when said forming structures exhibit sidewalls which are not oriented substantially perpendicular to the plane of the web, those portions of the sidewalls which correspond to the visible surface of the web are preferably provided with said pattern of surface aberrations. The importance of such sidewall preparation will, of course, increase as the degree of sidewall taper increases, since increased sidewall taper generally means a greater degree of sidewall visibility and hence a greater potential for specular reflection.

As will be appreciated by those skilled in the art, forming structures exhibiting tapered sidewalls, whether of integral or laminate construction, may have their entire visible surfaces subjected to a photoetching process to impart the desired pattern of surface aberrations thereto. Where laminar structures of the type generally illustrated in Figure 20 employ tapered sidewalls, the uppermost surface of all exposed laminae may be provided with the desired pattern of surface aberrations prior to assembly to ensure that those portions of the structure corresponding to the web's visible surface exhibit the desired pattern. Alternatively or additionally, the edges of the apertures may be scalloped to impart the desired surface aberrations to the tapered sidewalls of the resultant macroscopically expanded three-dimensional plastic webs.

While the preferred one-sided process disclosed in Figure 17 simultaneously imposes the pattern of surface aberrations and the macroscopic three-dimensional pattern of the forming structure on the web, it will be appreciated by those skilled in the art that these operations could be performed sequentially from the same side of the web. That is, the web could be caused to conform to a first forming structure exhibiting the desired pattern of surface aberrations, removed from said first forming structure and thereafter macroscopically expanded on a second three-dimensional forming structure. In the latter situation, said first forming structure could comprise a woven wire mesh screen exhibiting a knuckle pattern which satisfies the gloss minimization criteria herein set forth.

As was pointed out earlier herein, one-sided processes of the type generally illustrated in Figure 17 will work most effectively on films having a substantially uniform planar thickness of two mils (i.e., 0.05 mm) or less. The basic operational steps generally disclosed in the automated process of Figure 17 are substantially the same as the non-automated steps utilized to produce the webs disclosed in Figures 1-4, 5-8 and 9-12 from a substantially planar ribbon of one mil (i.e., 0.025 mm) thick opaque polyethylene film. On films having a substantially uniform planar thickness greater than 0.05 mm (two mils) it becomes difficult to transmit fine scale patterns of surface aberrations, which typically comprise protuberances, completely through the film's thickness with sufficient detail to yield the desired improvements in gloss reduction and tactile impression. Accordingly a two-sided process of the type generally disclosed in Figure 21 is usually preferred when dealing with relatively thick films. The basic operational steps generally described in the automated process of Figure 21 are substantially the same as the non-automated steps utilized to produce the web disclosed in Figures 13-16 from a substantially planar ribbon of 0.025 mm (one mil) thick opaque polyethylene film.

It must, of course, be recognized that in most instances the surface aberrations produced in webs formed by such a two-sided process comprise depressions rather than protuberances. Nonetheless the same basic criteria must be met by the resultant web to provide gloss minimization. Namely, the regularly spaced, microscopic pattern of surface aberrations must be sufficiently small that the pattern is not discernible to the naked eye when the perpendicular distance between the viewer's eye and the plane of the web is 30 cm or greater, each of said surface aberrations must be free of any planar areas which are large enough to inscribe a four mil (i.e., 0.1 mm) diameter circle, and each of said surface aberrations must be so spaced relative to all adjacent surface aberrations that the maximum diameter of any circle which can be inscribed on any planar surface intermediate said surface aberration and said adjacent surface aberrations is less than four mils (i.e., 0.1 mm).

In order to provide a cloth-like or fiber-like tactile impression in the resultant macroscopically expanded three-dimensional plastic web, it has been found that the surface aberrations in said web should exhibit an amplitude, i.e., the perpendicular distance from the bottom of said depression to the plane in which said depression originates, of at least 0.2 mils (i.e., 0.05 mm), and most preferably at least 0.3 mils (i.e., 0.0075 mm).

Experience has demonstrated that a more cloth-like or fiber-like tactile impression is perceived in macroscopically expanded three-dimensional plastic webs which meet the aforementioned amplitude criteria whether the surface aberrations comprise protuberances or depressions. This is believed to be due to the fact that in either case the surface of the web is divided into at least two distinct planes separated from one another by a distance of at least 0.2 mils (i.e., 0.005 mm). In the case of protuberances, it is the tops of the aberrations which contact the observer's skin, while in the case of depressions it is the planar surface in which said aberrations originate which contacts the observer's skin. Because said division is carried out in a fine microscopic pattern, it is believed that only the reduced area of contact with the uppermost surface of the web and not existence of the pattern is tactilely perceived. This seems consistent with the observation that the more cloth-like or fiber-like impression described herein is most readily perceived when macroscopically expanded three-dimensional plastic webs of the present invention are superposed on a substantially deformable substrate such as airfelt, sponge, cellulosic fibers, or any other material having generally similar deformation characteristics. It is believed that the deformable nature of the substrate prevents an observer touching the surface of the web from exerting a force sufficient to significantly deform or otherwise alter the surface characteristics of the web, thereby preserving the reduced area of contact with the observer's skin.

The two-sided process schematically illustrated in Figure 21 is identical to that pictorially illustrated in Figure 17 with three basic exceptions: first, freely rotatable lead-on idler roll 565 has been eliminated, and a suction roll 665 which is driven in conjunction with debossing/perforating cylinder 555 has been installed in its place; second, the web contacting surface of debossing/ perforating cylinder 555 need not be provided with a fine scale pattern of surface aberrations 160; and third, a hot air jet means 659 generally similar to hot air jet means 559 has been installed adjacent the surface of suction roll 665. Suction roll 665 is provided with a porous web contacting surface which, in a particularly preferred embodiment, comprises a fine mesh wire screen.

A stationary suction chamber (not shown) located interiorly of suction roll 665 permits suction to be applied to the roll contacting surface of the imperforate substantially planar plastic web 550 substantially throughout the area of contact therebetween. The imperforate substantially planar plastic web 550 is preferably heated to its softening temperature by hot air jet means 659, so that the suction applied by roll 665 and the mechanical pressure which may, if desired, be applied in the nip between suction roll 665 and debossing/perforating cylinder 555 have imparted the knuckle pattern exhibited by suction roll 665 to the suction roll contacting surface of the substantially planar web 550 by the time the web passes out of the nip.

By making certain that the pattern of surface aberrations thus imparted to the entire surface of the substantially planar plastic web 550 meets the criteria required for gloss elimination and improved tactile impression, as set forth earlier herein, the substantially planar web 550 may thereafter be processed, with minor exceptions, generally in accordance with the teachings of the aforementioned patent to Lucas et al. to provide a macroscopically expanded three-dimensional plastic web exhibiting a substantially non-glossy visible surface and a more cloth-like or fiber-like tactile impression.

The exceptions to the teachings of the Lucas et al. patent relate generally to a reduction in temperature of the plastic web 550 during the three-dimensional debossing and, if desired, aperturing operation. To avoid substantially washing out the pattern of surface aberrations imparted to the suction roll contacting surface of web 550 by suction cylinder 665, the temperature of the web is preferably not elevated substantially beyond its softening temperature during the subsequent debossing and perforating operations. Accordingly, somewhat higher suction levels may be required in the debossing and perforating portion of cylinder 555 to achieve the desired macroscopic expansion of the web than is the case for a higher temperature one-sided process of the type generally illustrated in Figure 17.

While any porous three-dimensional surface which satisfies the requirements for gloss elimination described earlier herein may be used as a web contacting surface on suction roll 665, the knuckle patterns of fine mesh wire screens have been found particularly suitable. In particular, for filament diameters between one and two mils (i.e., between 0.025 and 0.05 mm), screens having mesh counts ranging from 63 filaments per lineal cm by 63 filaments per lineal cm to 157 filaments per lineal cm by 157 filaments per lineal cm have been found operable, with screens having 100 filaments per lineal cm by 100 filaments per lineal cm being optimal. The larger filament diameters are generally operable with mesh counts at the lower end of the range, while the smaller filament diameters are generally operable with mesh counts at the upper end of the range. The weave pattern of the screen may be varied as desired, so long as the resultant pattern of surface aberrations produced in the web satisfies the gloss reduction and tactile impression criteria described earlier herein.

As will be appreciated by those skilled in the art, when the web contacting surface of suction roll 665 is a wire screen, the screen may be permanently secured to the periphery of the roll or fed in the form of a continuous belt across the web contacting surface of suction roll 665. In the latter embodiment, the screen may, if desired, serve as a carrier belt and remain in contact with debossing/perforating cylinder 555 through at least a portion of the web's trajectory. Both alternatives are schematically illustrated in Figures 3 and 4, respectively, of U.S. Patent 2,776,451 issued to Chavannes on January 8, 1957.

It will also be appreciated by those skilled in the art that the order in which the steps of the two-sided process generally disclosed in Figure 21 are carried out could be reversed, i.e., the web could be macroscopically expanded to its three-dimensional profile and thereafter brought in contact with a forming structure exhibiting the desired pattern of surface aberrations while said web is still being supported on said three-dimensional forming structure. The latter arrangement may find particular utility where the macroscopically expanded three-dimensional webs exhibit capillary networks having sidewalls oriented substantially perpendicular to the plane of said web.

As will be further appreciated by those skilled in the art, while the one-sided process generally described in connection with Figure 17 and the two-sided process generally described in conjunction with Figure 21 have dealt with converting substantially planar plastic webs into macroscopically expanded three-dimensional plastic webs, the present invention may be practiced with equal facility where resinous melts are involved.

A one-sided process of this type is schematically disclosed in Figure 22, wherein a resinous melt 550' is extruded by means of extruder 700 directly onto the surface of a debossing/perforating cylinder 555 exhibiting a microscopic pattern of surface aberrations about its periphery. Depending upon the proximity of the extruder 700 to the point of debossing and perforating and the temperature of the resinous melt 550' at said point, hot air jet means 559 may prove unnecessary.

An exemplary two-sided process employing a resinous melt is schematically disclosed in Figure 23. The process of Figure 23 is generally similar to the one-sided process of Figure 22, but suction roll 665 has been added to impart the desired pattern of surface aberrations to the suction roll contacting surface of the resultant plastic web. Accordingly it is not necessary to provide a microscopic pattern of surface aberrations about the periphery of debossing/perforating cylinder 555. It will, of course, be recognized by those skilled in the art that the extruder 700 could with equal facility be positioned to apply resinous melt 550' directly onto the surface of suction roll 665 for subsequent transfer to the surface of debossing/ perforating cylinder 555.

While particular embodiments of the present invention have been illustrated and described, it will be obvious to those skilled in the art that various changes and modifications can be made without departing from the spirit and scope of the invention. For example, the manufacturing processes generally disclosed in Figures 17 and 21-23 might be carried out by means of mechanical compression either solely or in combination with fluid pressure differentials, the one-sided and two-sided processes might be carried out in conjunction with one another to produce superposed patterns of surface aberrations, protuberances and depressions might be combined with one another in a single regularly spaced, microscopic pattern of surface aberrations, etc.

## Claims

1. A plastic web (30, 40) having a three dimensional macroscopic pattern and having a visible surface exhibiting microscopic surface aberrations (33, 43) which are not discernible to the naked eye when the perpendicular distance between the viewer's eye and the plane of said web is at least 30 cm, each of said surface aberrations being free of planar areas which are large enough to inscribe a 0.1 mm diameter circle, whereby light incident upon said visible surface is diffusely reflected into a multiplicity of directions by said surface aberrations to reduce the gloss on said surface, characterised in that, said surface aberrations are regularly sized and are disposed in a regularly spaced pattern over substantially all of said visible surface, said aberration size being measured in the plane in which said surface aberrations originate, said spaced pattern being such that the maximum diameter of any circle which can be inscribed on any planar surface of the web intermediate adjacent surface aberrations is less than 0.1 mm, the minimum average amplitude of said surface aberrations being 0.005 mm, as measured perpendicular to and from the surface in which said surface aberrations originate, the maximum dimension of any said surface aberrations being less than 0.15 mm as measured substantially perpendicular to the amplitude of said surface aberrations, in the plane in which said surface aberrations originate, whereby said visible surface is rendered substantially non glossy.

2. A web according to Claim 1, wherein at least a portion of said surface aberrations comprise protuberances (33) projecting generally outwardly from the surface (31) of said web.

3. A web according to Claim 1, wherein at least a portion of said surface aberrations comprise depressions (43) projecting generally inwardly from the surface (41) of said web.

4. A web according to Claim 3, wherein said surface aberrations are disposed in a pattern corresponding to that of the knuckles of a woven mesh support structure.

5. A web according to Claim 4, wherein the pattern of said woven mesh support structure is a square weave of 0.025-0.05 mm diameter filaments having a mesh count in the range 63-157 filaments per lineal cm in each direction.

6. A web according to any one of Claims 1-5, wherein said surface aberrations have a minimum amplitude of at least 0.01 mm, preferably at least 0.015 mm.

7. A web according to Claim 6, wherein the amplitude of each of said surface aberrations, as measured perpendicular to the surface in which said surface aberrations originates, is within ±20%, preferably ±10% of the average value of the amplitude for all adjacent surface aberrations.

## Patentansprüche

1. Kunststoffbahn (30, 40) mit einem dreidimensionalen makroskopischen Muster und einer sichtbaren Oberfläche, die mikroskopische Oberflächen-Aberrationen (33, 43) aufweist, die mit dem bloßen Auge nicht erkennbar sind, wenn der lotrechte Abstand zwischen dem Auge des Betrachters und der Ebene dieser Bahn mindestens 30 cm beträgt, wobei jede der Oberflächen-Aberrationen frei von ebenen Bereichen sind, die groß genug sind, um einen Kreis mit 0,1 mm Durchmesser einzubeschrieben, wodurch der Lichteinfall auf die sichtbare Oberfläche durch diese Oberfläche-Aberrationen zur Verhinderung des Glänzens dieser Oberfläche diffus in eine Vielzahl von Richtungen reflektiert wird, dadurch gekennzeichnet, daß diese Oberflächen-Aberrationen eine regelmäßige Größe haben und in einem Muster mit regelmäßigen Abständen über im wesentlichen die gesamte sichtbare Oberfläche angeordnet sind, wobei die Aberrations-Größe in der Ebene gemessen wird, in der diese Oberflächen-Aberrationen beginnen, wobei das Abstandsmuster so ist, daß der größte Durchmesser eines Kreises, der auf irgendeiner ebenen Oberfläche der Bahn zwischen angrenzenden Oberflächen-Aberrationen einbeschrieben werden kann, weniger als 0,1 mm beträgt, die kleinste mittlere Amplitude dieser Oberflächen-Aberrationen 0,005 mm, gemessen senkrecht zu und von der Oberfläche, in welcher diese Oberflächen-Aberrationen beginnen, beträgt, die größte Dimension irgendeiner dieser Oberflächen-Aberrationen kleiner als 0,15 mm, gemessen im wesentlichen senkrecht zu der Amplitude der genannten Oberfläche-Aberrationen in der Ebene ist, in welcher diese Oberflächen-Aberrationen beginnen, wodurch diese sichtbare Oberfläche im wesentlichen nicht glänzend gemacht wird.

2. Bahn nach Anspruch 1, worin mindestens ein Teil dieser Oberflächen-Aberrationen Protuberanzen (33) aufweist, die sich allgemeinerweise nach außen von der Oberfläche (31) dieser Bahn erstrecken.

3. Bahn nach Anspruch 1, worin mindestens ein Teil dieser Oberflächen-Aberrationen Vertiefungen (43) aufweist, die allgemeinerweise sich nach innen von der Oberfläche (41) dieser Bahn erstrecken.

4. Bahn nach Anspruch 3, worin die Oberflächen-Aberrationen in einem Muster angeordnet sind, das demjenigen von Buckeln einer gewebten Maschenträgerstruktur entspricht.

5. Bahn nach Anspruch 4, worin das Muster dieser gewebten Maschenträgerstruktur eine rechtwinklige Bindung von Fäden mit einem Durchmesser von 0,025-0,05 ist, die eine Maschenzahl im Bereich von 63 bis 157 Fäden je linearem Zentimeter in jeder Richtung aufweist.

6. Bahn nach einem der Ansprüche 1 bis 5, worin die Oberflächen-Aberrationen eine Mindestamplitude von mindestens 0,01 mm, vorzugsweise mindestens 0,015 mm aufweisen.

7. Bahn nach Anspruch 6, worin die Amplitude jeder der Oberflächen-Aberrationen, lotrecht zur Oberfläche gemessen, von der die Oberflächen-Aberrationen ausgehen, innerhalb ± 20%, vorzugsweise ± 10%, des Durchschnittswertes der Amplitude für alle benachbarten Oberflächen-Aberrationen beträgt.

## Revendications

1. Une nappe plastique (30, 40) ayant un dessin tridimensionnel macroscopique et ayant une surface visible présentant des irrégularités de surface microscopiques (33, 43) qui ne peuvent pas être discernées à l'oeil nu lorsque la distance perpendiculaire entre l'oeil de l'observateur et le plan de ladite nappe est au moins de 30 cm, chacune desdites irrégularités de surface étant exemptes de zones planes qui sont suffisamment grandes pour s'inscrire dans un cercle de 0,1 mm de diamètre, de telle sorte que la lumière incidente sur ladite surface visible est réfléchie de façon diffuse en une multiplicité de directions par lesdites irrégularités de surface afin de réduire le brillant sur ladite surface, caractérisé en ce que lesdites irrégularités de surface sont régulièrement dimensionnées et sont disposées suivant un dessin espacé régulièrement sur pratiquement toute ladite surface visible, ladite dimension des irrégularités étant mesurée dans le plan dans lequel lesdites irrégularités de surface débutent, ledit dessin espacé étant tel que le diamètre maximal de tout cercle qui peut être inscrit sur toute surface plane de la nappe entre les irrégularités de surface adjacentes est inférieur à 0,1 mm, l'amplitude moyenne minimale desdites irrégularités de surface étant de 0,005 mm, comme mesuré perpendiculairement à et à partir de la surface de laquelle lesdites irrégularités de surface débutent, la dimension maximale de toute irrégularité de surface étant inférieure à 0,15 mm, comme mesuré essentiellement perpendiculairement à l'amplitude desdites irrégularités de surface dans le plan dans lequel lesdites irrégularités de surface débutent, si bien que ladite surface visible est rendue essentiellement non brillante.

2. Une nappe selon la revendication 1, caractérisée en ce qu'au moins une partie desdites irrégularités de surface comporte des protubérances (33) qui font saillie généralement vers l'extérieur à partir de la surface (31) de ladite nappe.

3. Une nappe selon la revendication 1, caractérisée en ce qu'au moins une partie desdites irrégularités de surface comporte des dépressions (43) qui font saillie généralement vers l'intérieur à partir de la surface (41) de ladite nappe.

4. Une nappe selon la revendication 3, caractérisée en ce que lesdites irrégularités de surface sont disposées suivant un dessin correspondant à celui des bossages de la structure de support à maille tissée.

5. Une nappe selon la revendication 4, caractérisée en ce que le dessin de ladite structure de support à maille tissée est une chaîne carrée de filaments de 0.025-0.05 mm de diamètre ayant un nombre de maille de l'ordre de 63-157 filaments par cm linéaire dans chaque direction.

6. Une nappe selon l'une quelconque des revendications 1-5, caractérisée en ce que lesdites irrégularités de surface ont une amplitude minimale d'au moins 0.01 mm, et de préférence au moins 0,015 mm.

7. Une nappe selon la revendication 6, caractérisée en ce que l'amplitude de chacune desdites irrégularités de surface mesurée perpendiculairement à la surface dans laquelle débute ladite irrégularité de surface, est de l'ordre de ±20%, et de préférence de ±10% de la valeur moyenne de l'amplitude pour toutes les irrégularités de surface adjacentes.
